Europäisches Patentamt

(19) European Patent Office

Office européen des brevets

(11) Numéro de publication: **0 123 566**

**B1**

(12) **FASCICULE DE BREVET EUROPÉEN**

(45) Date de publication du fascicule du brevet:
08.06.88

(51) Int. Cl.⁴: **A 61 L 9/16,** A 61 M 16/00

(21) Numéro de dépôt: **84400128.9**

(22) Date de dépôt: **20.01.84**

(54) Procédé de production d'air stérile pour usage médical et installation pour la mise en oeuvre de ce procédé.

(30) Priorité: **26.01.83 FR 8301156**

(43) Date de publication de la demande:
**31.10.84 Bulletin 84/44**

(45) Mention de la délivrance du brevet:
**08.06.88 Bulletin 88/23**

(84) Etats contractants désignés:
**AT BE CH DE FR GB IT LI LU NL SE**

(56) Documents cité:
**FR-A-2 102 481**
**FR-A-2 431 660**
**FR-A-2 493 705**
**FR-A-2 504 024**
**US-A-3 239 305**
**US-A-3 469 934**
**US-A-3 966 407**
**US-A-4 231 768**

Le dossier contient des informations techniques présentées postérieurement au dépôt de la demande et ne figurant pas dans le présent fascicule.

(73) Titulaire: **COMPAGNIE FRANCAISE DE PRODUITS OXYGENES Société anonyme, 1, rue Bayard, F-75008 Paris (FR)**

(72) Inventeur: **Delachapelle, Henri, 39bis boulevard Robert Schumann, F-44000 Nantes (FR)**

(74) Mandataire: **Boutin, Antoine, CABINET PIERRE LOYER 77, rue Boissière, F-75116 Paris (FR)**

EP 0 123 566 B1

LIBER, STOCKHOLM 1988

## Description

L'invention concerne un procédé de production d'air médical destiné à être administré aux malades en cours de réanimation.

Cet air médical doit autant que possible être exempt de toute impureté et de tout germe.

Les techniques classiques de traitement de l'air atmosphérique (cf brevet FR 2 493 705 et brevet US 3 469 934) ne permettent pas d'obtenir un air de qualité suffisante pour qu'il soit administré à des malades.

Des tentatives pour transformer l'air atmosphérique en air médical ont déjà été faites (cf brevet US 4 231 768) mais elles n'ont pas permis jusqu'à présent des résultats satisfaisants. Ainsi, par ce brevet US n° 4 231 768 est connue une installation de purification de l'air à partir d'air atmosphérique comprimé, ladite installation comprenant notamment un filtre coalescent suivi d'un dispositif de dessication, d'un absorbeur de gaz, d'un dispositif de filtration des poussières et un dispositif d'élimination des bactéries. Mais en raison du fait que le compresseur utilisé est un compresseur à pistons lubrifiés par huile, une telle installation nécessite une série de filtres de déshuilage de l'air, ce qui implique des interventions de maintenance fréquentes pour nettoyer ou remplacer des filtres à huile qui s'encrassent rapidement. Une telle installation est donc incapable de fonctionner en continu, sans interruption, ce qui est un impératif pour une station de production hospitalière. C'est la raison pour laquelle l'air médical actuellement utilisé est produit par mélange d'azote et d'oxygène pur. Le prix de revient de cet air est relativement élevé.

Le procédé selon l'invention permet de produire de l'air stérile pour un usage médical à partir de l'air atmosphérique, l'air ainsi produit ayant un prix de revient sensiblement diminué par rapport à l'air stérile obtenu par le mélange d'azote et d'oxygène purs.

Selon l'invention, il est proposé un procédé de production d'air stérile sec pour usage médical à partir d'air atmosphérique comprimé, puis soumis aux étapes de traitement suivantes:
- une étape de condensation de l'eau,
- une étape de filtration des poussières jusqu'au stade du μm,
- une étape de dessiccation,
- une étape d'élimination des gaz,
- une deuxième étape de filtration des poussières jusqu'au stade du μm,
- une étape d'élimination bactérienne,
caractérisé en ce que l'air est comprimé au moyen d'au moins un compresseur à pistons secs pour l'étape de dessication et l'étape d'élimination des gaz, on utilise deux filtres à adsorption-désorption, dont le produit actif est de même nature, le filtre éliminateur des gaz étant disposé en aval du filtre dessiccateur et comportant une plus grande quantité de produit actif que ce dernier.

Avantageusement, le filtre éliminateur des gaz contient environ 50 % de produit actif de plus que le dessiccateur.

Selon une autre caractéristique de l'invention, pour l'étape de filtration bactérienne, on utilise trois filtres à coalescence à seuil de filtration décroissant jusqu'à 0,01 μm, suivis d'un stériliseur à froid.

Lorsque l'air ambiant contient de l'oxyde de carbone CO, on prévoit, dans la première séquence, entre l'opération d'élimination des poussières et celle d'élimination de la vapeur d'eau, une étape supplémentaire de transformation de l'oxyde de carbone CO en gaz carbonique $CO_2$.

L'invention concerne également un dispositif pour la mise en oeuvre du procédé ci-dessus, comprenant:
- au moins un compresseur sec,
- un réservoir tampon assurant la régulation du débit du compresseur et une élimination de l'eau par condensation due au refroidissement,
- un premier filtre à poussières micronique,
- un dessiccateur,
- un dessulfureur,
- un second filtre à poussières,
- un préfiltre microbiologique,
- un séparateur microbiologique,
- un désodoriseur microbiologique,
- un stériliseur à froid.

Lorsque l'air initial est chargé d'oxyde de carbone CO, l'installation comporte en outre un appareil à catalyse qui transforme l'oxyde de carbone CO en gaz carbonique $CO_2$, suivi d'un échangeur-condenseur. Ces deux appareils sont disposés à la sortie du premier filtre à poussières avant le dessiccateur.

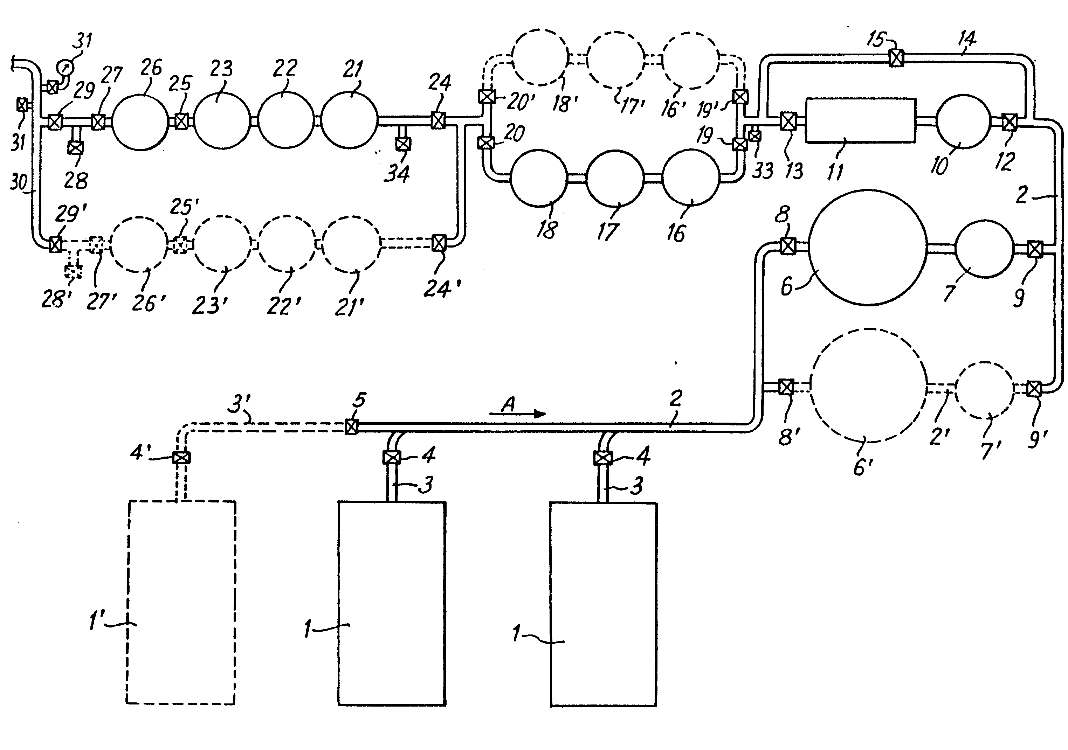

D'autres caractéristiques de l'invention ressortiront de la description ci-après d'un exemple de réalisation d'une installation conforme à l'invention en référence au dessin annexé qui représente le schéma d'une telle installation.

Sur ce schéma, le sens de circulation de l'air est indiqué par la flèche A.

L'installation comporte cinq catégories d'appareils correspondant à cinq stades critiques du procédé selon l'invention, à savoir:

1 - La compression de l'air.
2 - Le traitement préparatoire.
3 - Le traitement de l'oxyde de carbone.
4 - La dessiccation et l'élimination des gaz nocifs.
5 - L'hyperfiltration et la stérilisation.

### 1 - La compression de l'air.

Cet étage comporte deux compresseurs 1, du type à pistons secs vendus par la demanderesse sous la référence DTI 1818. Les compresseurs de ce genre ne nécessitent ni graissage, ni lubrification, du fait que leurs bielles et les axes de leurs pistons sont montés sur des roulements graissés à vie, tandis que leurs segments sont

fabriqués dans un matériau autolubrifiant à base de PTFE.

De ce fait, l'air comprimé est exempt d'huile provenant du fonctionnement du compresseur lui-même.

Un compresseur additionnel 1', représenté en traits pointillé sur le schéma, est installé en parallèle avec les deux compresseurs 1. Il sert de compresseur de secours lorsque des interventions de maintenance ou de réparation sont effectuées sur l'un ou l'autre des deux compresseurs principaux 1.

Les compresseurs 1 sont reliés à une canalisation principale 2 de circulation de l'air par deux dérivations 3, comportant chacune une vanne 4.

De même, le compresseur de secours 1' est relié à la canalisation 2 par une dérivation 3', comportant une vanne 4'.

Une deuxième vanne 5 est disposée à l'embranchement de la dérivation 3' avec la canalisation 2.

## 2 - Le traitement préparatoire.

La canalisation 2 amène l'air comprimé dans un réservoir tampon 6, qui a pour fonction d'éliminer les pulsations de l'air comprimé du débit des compresseurs et d'extraire partiellement l'eau contenue dans l'air. En effet, l'air échauffé et comprimé par le compresseur se refroidit en se détendant dans le réservoir 6, ce qui provoque la condensation sur les parois du réservoir des gouttelettes d'eau contenues dans l'air.

Derrière le réservoir 6 est implanté un filtre de séparation micronique 7, dont le rôle est de filtrer les poussières contenues dans l'air jusqu'au stade du µm.

Des filtres convenables à cet effet sont par exemple des filtres à coalescence connus, dont l'élément filtrant est une cartouche en alumine vitrifiée à 1.100°C. De tels filtres sont vendus par la demanderesse sous la référence SPM.

Une vanne d'entrée 8 et une vanne de sortie 9 permettent d'isoler cette partie de l'installation. De même que pour les compresseurs, un équipement de secours parallèle (représenté en traits pointillés) est prévu, qui comporte, sur une dérivation 2', un réservoir 6', un filtre de séparation micronique 7' et deux vannes 8', 9'.

## 3 - Le traitement de l'oxyde de carbone.

A la sortie du filtre 7, l'air est amené à un dispositif 10 de transformation de l'oxyde de carbone CO en gaz carbonique $CO_2$ par catalyse.

A cet effet, on fait passer l'air dans un appareil de catalyse, comportant un oxydant métallique qui, sous l'effet d'une température de 120° à 130°C environ, obtenue par une résistance électrique, est chargé d'assurer l'oxydation complète de l'oxyde de carbone en vue de sa transformation en gaz carbonique $CO_2$.

On peut avantageusement utiliser comme oxydant métallique, des pastilles d'acier ou de matière réfractaire plaquées de platine. De préférence, ces pastilles ont une surface repésentant des aspérités pour augmenter la surface d'échange. Un tel dispositif est vendu par la demanderesse sous la marque déposée CATALAIR.

L'air ainsi traité ayant été porté à une température d'environ 120° à 130°C par chauffage, il convient de le ramener à la température ambiante avant de lui faire subir le traitement de dessiccation et d'extraction des gaz prévu à l'étape suivante.

Pour ce faire, on fait passer l'air refoulé par l'appareil 10 dans un échangeur-condenseur ou refroidisseur 11, de type connu, qui de préférence, a une importante surface d'échange. Un refroidisseur convenable est, par exemple, constitué par un tube de cuivre disposé en zigzag sur lequel sont soudées des ailettes d'aluminium de grande dimension, l'ensemble étant associé à un ventilateur.

Un tel appareil est vendu par la demanderesse sous la dénomination commerciale ZED.

En variante, le dispositif de catalyse 10 et son échangeur 11, peuvent être disposés entre le dessiccateur 16 et le dessulfureur 17, qui seront décrits plus loin.

Après son passage dans ledit dessulfureur 17, l'air en traitement est totalement débarrassé du $CO_2$. Si pour certaines applications il est désirable que l'air traité contienne une petite quantité de $CO_2$, il est judicieux de placer le dispositif de catalyse 10 et son échangeur 11 en avant du dessulfureur 17 et avant la série des filtres à coalescence 21, 22, 23, de l'étape de filtration bactérienne qui sera décrite ultérieurement.

Ainsi, le $CO_2$ contenu dans l'air atmosphérique est éliminé par le dessulfureur, alors que l'apport de $CO_2$ additionnel requis est fourni par le positif de catalyse 10 et son échangeur 11 à partir du CO contenu dans l'air atmosphérique.

De préférence, le dispositif de catalyse 10 et son échangeur sont placés entre le deuxième filtre de séparation des poussières 18 et le premier filtre 21 de l'étape de filtration bactérienne.

Deux vannes 12 et 13 sont disposées à l'entrée du dispositif de transformation de l'oxyde de carbone 10 et à la sortie de l'échangeur condenseur 11.

D'autre part, une dérivation 14 de la canalisation 2, munie d'une vanne 15, permet d'amener l'air directement à l'étage de dessiccation et d'extraction des gaz lorsque cet air ne contient pas d'oxyde de carbone.

Bien que cela ne soit pas représenté sur le schéma, il peut également être prévu un circuit de secours parallèle, comportant un catalyseur et un refroidisseur.

## 4 - La dessiccation et l'extraction des gaz.

A la sortie du refroidisseur 11, l'air est soumis à une dessiccation profonde dans un dessiccateur 16, fonctionnant par adsorption-désorption. Le produit actif contenu dans l'appareil est un combiné d'oxydes métalliques avec des silicates de potassium, de sodium et de magnésium fabriqué par la société GRACE GMBH, sous la référence 562.

Le dessiccateur 16 est constitué par deux bouteilles sous pression, dont l'une est en service pendant que l'autre est en régénération. Un dispositif permet d'inverser périodiquement et automatiquement le fonctionnement des bouteilles. Pendant la phase de régénération (désorption), la bouteille concernée est chauffée par une résistance électrique portée à une température d'environ 180°C. Les molécules d'eau précédemment adsorbées sont libérées et sont entraînées vers une purge par un très léger soufflage d'air sec en provenance de la bouteille en service.

Un tel appareil est vendu par la demanderesse sous la dénomination commerciale DESSICAIR.

Cette opération de dessiccation est nécessaire pour éliminer de l'air l'eau sous forme de vapeur qui n'a pas été extraite par condensation dans le réservoir 6 au stade du traitement préparatoire.

A la suite de l'appareil de dessiccation 16, est disposé un appareil d'élimination de gaz 17, appelé généralement dessulfureur, dont le rôle est de soustraire l'anhydre sulfureux, l'hydrogène sulfureux, le péroxyde d'azote, le gaz carbonique, les vapeurs d'hydrocarbures, le fluor, le chlore, les mercaptans, le tétraéthyle ou tétraméthyle de plomb et tous les autres gaz indésirables généralement présents dans l'atmosphère des villes.

Le dessulfureur 17 est un appareil semblable au dessiccateur 16. Il fonctionne selon le même principe d'asorption-désorption et son élément actif est également un composé d'oxydes métalliques et de silicate de potassium, de sodium et de magnésium. Mais, d'une part, les proportions de la composition du produit sont différentes et, d'autre part, la quantité de produit actif est plus importante dans le dessulfureur que dans le dessiccateur.

Pour le dessulfureur, on peut avantageusement utiliser le produit fabriqué sous la référence 542 par la société GRACE GMBH dans une quantité supérieure d'environ 50 % à celle du produit contenu dans le dessiccateur.

L'adsorbant du dessulfureur 17 désodorise l'air en même temps qu'il en élimine les gaz.

La désorption s'effectue à une température de 220°C au lieu de 180°C dans le dessiccateur 16.

A la sortie du dessulfureur 17 est disposé un deuxième filtre de séparation micronique 18, identique au filtre 7.

La fonction de ce deuxième filtre à poussières est de retenir les poussières qui pourraient provenir de l'érosion des adsorbants utilisés dans les appareils 16 et 17.

Deux vannes 19 et 20 sont disposées respectivement en amont de l'appareil de dessiccation 16 et en aval du deuxième filtre 18.

Une installation de secours est disposée en parallèle (traits pointillés), elle comporte également un dessiccateur 16', un dessulfureur 17', un filtre 18' et deux vannes 19' et 20'.

A ce niveau de l'installation, l'air est sec et débarrassé de ses particules d'origine minérale indésirables. Il reste à le débarrasser de ses particules d'origine biologique (virus, microbes) dans la séquence d'hyperfiltration et de stérilisation.

## 5 - L'hyperfiltration et la stérilisation.

Par hyperfiltration, on entend la filtration jusqu'à un seuil de l'ordre du centième de µm.

A cet effet, on dispose une batterie de trois filtres 21, 22, 23 à coalescence de type connu, dont l'élément filtrant est constitué par une nappe de fibres de verre au borosilicate recouverte par une gaine en matière plastique poreuse, avec une densité de fibres de verre déterminant un seuil de filtration décroissant d'un filtre à l'autre.

Ainsi, dans le premier filtre 21, le seuil de filtration est de 0,6 µm, tandis que dans le second et le troisième filtres 22, 23, le seuil est de 0,01 µm.

Le troisième filtre 23 comporte en outre un dispositif d'absorption au carbone actif qui, outre son action de rétention des bactéries, procure une action désodorisante qui complémente celle du dessulfureur 17 précédemment décrit.

Des filtres à coalescence, tels que les fitres 21, 22 et 23, sont vendus par la demanderesse sous les références PFM, SMB et DSO respectivement.

Deux vannes 24 et 25 sont disposées respectivement à l'entrée et à la sortie de l'étage d'hyperfiltration de l'installation.

Un stérilisateur à froid, connu en soi, 26 est disposé en amont de la vanne 24. Le stérilisateur comporte une cartouche en fibres de verre au borosilicate, ayant reçu un traitement hydrophobe, tenue dans une armature en acier inoxydable. Cette cartouche est logée dans un corps en acier inoxydable. Un tel stériliseur est vendu par la demanderesse sous la référence STR.

Une vanne 27 est disposée à la sortie du stériliseur 26. De la sorte, en fermant les vannes 25 et 26, on peut isoler le stériliseur, qui peut ainsi recevoir un traitement de stérilisation périodique.

En aval de la vanne 27, on trouve successivement une prise de test 28, une vanne de fermeture générale 29, la canalisation de sortie 30 avec un manomètre 31 et un manostat de sécurité 32.

La prise de test permet de contrôler la pureté de l'air à la sortie du stériliseur 26 et donc l'efficacité de l'installation.

Le manostat 32 arrête automatiquement le fonctionnement de l'installation lorsque la pression délivrée dans la canalisation de sortie 30 tombe à un niveau prédéterminé.

Des prises de tests 33 et 34 analogues à la prise de test 28 sont placées respectivement à la sortie de l'échangeur-condenseur 11 pour contrôler le bon fonctionnement du catalyseur 10 et avant le filtre 21 pour contrôler le bon fonctionnement de l'étage de dessiccation et dessulfuration.

De même que précédemment, l'étage d'hyperfiltration et de stérilisation est doublé par un étage de secours monté en parallèle, qui comporte également trois filtres 21', 22', 23', un stériliseur 26', les vannes 24', 25', 27', 29' et la prise de test 28'.

De façon classique, la canalisation de sortie 30 se branche sur le réseau de distribution d'air de l'hôpital ou de la clinique qu'il équipe.

De façon avantageuse, la canalisation de sortie 30 peut comporter une installation de soutirage (non représentée) permettant la mise en bouteille et le stockage d'air épuré.

Dans l'installation qui vient d'être décrite, le dispositif de traitement de l'oxyde de carbone 10 est implanté entre le premier filtre micronique 7 et l'installation de dessiccation 16.

Selon une variante avantageuse, on peut implanter cet appareil à la sortie des compresseurs 7, avant le réservoir 6. On utilise ainsi la chaleur de l'air sortant des compresseurs ce qui évite, ou diminue sensiblement, l'apport de calories nécessaire à l'action de catalyse.

Selon une autre variante de réalisation avantageuse du procédé selon l'invention, on réalise la filtration avec une faible vitesse de passage de l'air dans tous les filtres jusqu'au deuxième filtre de coalescence 22 de l'étage de filtration bactérienne, puis on accélère cette vitesse de passage de l'air dans le troisième filtre 23 et dans le stériliseur 26.

De préférence, dans ce cas, on place le filtre qui comporte un dispositif d'absorption au charbon actif en seconde position au lieu de la troisième.

Ceci permet d'éviter un encrassement trop rapide des éléments filtrants disposés en amont du troisième filtre à coalescence de l'étage de filtration bactérienne et en conséquence de réaliser une préfiltration plus efficace que lorsque la vitesse est constante sur toute la chaîne de filtration.

En second lieu, cette manière de procéder assure un contact plus long de l'air en traitement avec la partie désodorisante du deuxième filtre à coalescence de l'étage de filtration bactérienne, donc procure une désodorisation plus profonde.

Enfin, le fait d'utiliser une faible vitesse de passage de l'air dans la majeure partie de l'installation diminue très sensiblement les pertes de charge dans les filtres.

De préférence, la vitesse de passage de l'air dans le dernier filtre à coalescence 23 et dans le stériliseur 26 est une fois et demie à trois fois plus élevée que dans le reste de l'installation.

Un moyen simple d'obtenir une faible vitesse de passage de l'air dans les filtres situés en amont du dernier filtre à coalescence 23 et du stériliseur 26 et par conséquent, une accélération de la vitesse dans ces deux derniers appareils est d'utiliser des filtres à surface de passage surdimensionnée pour tous les appareils précédant les deux derniers.

**Revendications**

1. Procédé de production d'air stérile sec pour usage médical à partir d'air atmosphérique comprimé, puis soumis aux étapes de traitement suivantes:
- une étape de condensation de l'eau,
- une étape de filtration des poussières jusqu'au stade du μm,
- une étape de dessiccation,
- une étape d'élimination des gaz,
- une deuxième étape de filtration des poussières, jusqu'au stade du μm,
- une étape d'élimination des bactéries, caractérisé en ce que l'air est comprimé au moyen d'au moins un compresseur à pistons secs et en ce que, pour l'étape de dessiccation et l'étape d'élimination des gaz, on utilise deux filtres à adsorption-désorption dont le produit actif est de même nature, le filtre éliminateur des gaz étant disposé en aval du filtre dessiccateur et comportant une plus grande quantité de produit actif que ce dernier.

2. Procédé selon la revendication 1, caractérisé en ce que le filtre éliminateur des gaz contient environ 50 % de produit actif de plus que le filtre dessiccateur.

3. Procédé selon l'une quelconque des revendications 1 à 3, caractérisé en ce que pour l'étape d'élimination des bactéries, on utilise une série de trois filtres à coalescence à seuil de filtration décroissant jusqu'à 0,01 μm, suivie d'un stériliseur à froid.

4. Procédé selon l'une quelconque des revendications 1 à 3, caractérisé en ce que l'on réalise la filtration avec une faible vitesse de passage de l'air dans tous les filtres jusqu'au deuxième filtre à coalescence de l'étape de filtration bactérienne, puis on accélère la vitesse de passage de l'air dans le dernier filtre à coalescence de ladite étape et dans le stériliseur.

5. Procédé selon la revendication 4, caractérisé en ce que la vitesse de passage dans le dernier filtre à coalescence de l'étape de filtration bactérienne et dans le stériliseur est une fois et demie à trois fois supérieure à la vitesse de passage dans les autres appareils de l'installation.

6. Installation pour la production d'air stérile par le procédé selon l'une quelconque des revendications 1 à 5, caractérisée en ce qu'elle comporte successivement:
- au moins un compresseur à pistons secs (1),

- au moins un réservoir (b) assurant la régulation du débit du compresseur (1) et l'extraction de l'eau par condensation,
- un premier filtre de séparation des poussières (7),
- un dessiccateur par adsorption-désorption (16),
- un appareil d'élimination des gaz par adsorption-désorption (17),
- un deuxième filtre de séparation des poussières (18),
- une série de trois filtres à coalescence (21, 22, 23) à seuil de filtration s'abaissant progressivement jusqu'à 0,01 µm,
- un stériliseur à froid (26).

7. Installation selon la revendication 6, caractérisée en ce que le dessiccateur (16) et l'appareil d'élimination des gaz (17) comportent comme produit actif, un composé d'oxydes métalliques avec des silicates de potassium, de sodium et de magnésium, la quantité de produit actif contenu dans l'appareil d'élimination des gaz étant plus grande que dans le dessiccateur.

8. Installation selon la revendication 7, dans laquelle l'appareil d'élimination des gaz (17) comporte 50 % de produit actif de plus que le dessiccateur (16).

9. Installation selon l'une quelconque des revendications 6 à 8, caractérisée en ce que la série des filtres à coalescence (21, 22, 23) de l'étape d'élimination des bactéries ont un élément filtrant constitué par des fibres de verre au borosilicate revêtues d'une gaine en matière plastique poreuse.

10. Installation selon la revendication 9, caractérisée en ce que le dernier filtre (23) de la série comporte en outre un dispositif d'absorption au charbon actif.

11. Installation selon l'une quelconque des revendications 6 à 10, caractérisée en ce que le stériliseur à froid (26) a un élément filtrant constitué par une cartouche de fibres de verre au borosilicate ayant reçu un traitement hydrophobe, tenue dans une armature en acier inoxydable.

12. Installation selon l'une quelconque des revendications 6 à 11, caractérisée en ce qu'elle comporte en amont de l'appareil d'élimination des gaz (17), un dispositif de transformation de l'oxyde de carbone CO en gaz carbonique $CO_2$ (10, 11).

13. Installation selon la revendication 12, caractérisée en ce que le dispositif de transformation du CO en $CO_2$ (10, 11) est disposé en amont du dessiccateur (16).

14. Installation selon la revendication 12, caractérisée en ce que le dispositif de transformation du CO en $CO_2$ (10, 11) est disposé entre le compresseur (1) et le réservoir (7).

15. Installation selon l'une quelconque des revendications 6 à 11, caractérisée en ce qu'elle comporte en aval de l'appareil d'élimination des gaz (17) un dispositif de transformation de l'oxyde de carbone CO et gaz carbonique $CO_2$ (10, 11).

16. Installation selon la revendication 15, caractérisée en ce que le dispositif de transformation de l'oxyde de carbone CO en gaz carbonique $CO_2$ (10, 11) est placé entre le deuxième filtre de séparation des poussières (18) et le premier filtre à coalescence (21) de l'étape de filtration bactérienne.

17. Installation selon l'une quelconque des revendications 12 à 16, caractérisée en ce que le dispositif de transformation du CO en $CO_2$ comprend un appareil à catalyse (10) utilisant un oxydant métallique à une température de 120 à 130°C associé à un échangeur-condenseur (11).

18. Installation selon la revendication 17, caractérisée en ce que l'oxydant métallique est constitué par des pastilles métalliques ou en matière réfractaire, plaquées de platine.

19. Installation selon la revendication 18, caractérisée en ce que les pastilles ont une surface représentant des aspérités.

20. Installation selon l'une quelconque des revendications 6 à 19, caractérisée en ce que l'élément filtrant des filtres à poussières (7, 18) est une cartouche en alumine vitrifiée à 1.100°C.

21. Installation selon l'une quelconque des revendications 6 à 9 et 11 à 20, caractérisée en ce que le deuxième filtre à coalescence (22) de l'étape de filtration bactérienne comporte un dispositif d'absorption au charbon actif.

22. Installation selon l'une quelconque des revendications 6 à 21, caractérisée en ce que les filtres situés en amont du dernier filtre à coalescence (23) de l'étape de filtration bactérienne ont une surface de passage surdimensionnée.

**Patentansprüche**

1. Verfahren zur Erzeugung steriler Trockenluft für medizinische Zwecke ausgehend von atmosphärischer Druckluft, die folgenden Behandlungsschritten unterzogen wird:
- Kondensierung des Wassers
- Staubfilterung bis auf eine Korngröße von µm
- Trocknung
- Gasabscheidung
- zweite Staubfilterung bis auf eine Korngröße von µm
- und Bakterienabscheidung,
dadurch gekennzeichnet, daß die Druckluft mittels eines Trockenlaufkompressors verdichtet wird und daß für die Trocknung und Gasabscheidung zwei Adsorptions-Desorptionsfilter mit gleichartigem Aktivstoff verwendet werden, wobei der Gasabscheidungsfilter nach dem Trocknungsfilter angeordnet ist und eine größere Menge Aktivstoff enthält.

2. Verfahren nach Anspruch 1, dadurch gekennzeichnet, daß der Gasabscheidungsfilter ungefähr 50 % mehr Aktivstoff als der Trockungsfilter enthält.

3. Verfahren nach einem der Ansprüche 1 oder 2, dadurch gekennzeichnet, daß für die Bakterienabscheidung drei in Serie angeordnete Koaleszenzfilter mit abnehmender Filterschwelle bis zu 0,01 μm und ein nachgeschalteter Kaltsterilisator verwendet wird.

4. Verfahren nach einem der Ansprüche 1 bis 3, dadurch gekennzeichnet, daß die Filterung bis zum zweiten Koaleszenzfilter der Bakterienabscheidungsphase mit geringer Luftströmung erfolgt, die dann im letzten Koaleszenzfilter der o. a. Phase und im Sterilisator beschleunigt wird.

5. Verfahren nach Anspruch 4, dadurch gekennzeichnet, daß die Luftströmungsgeschwindigkeit im letzten Koaleszenzfilter der Bakterienabscheidungsphase und im Sterilisator eineinhalb bis dreimal so hoch wie die Luftströmungsgeschwindigkeit in den übrigen Teilen der Anlage ist.

6. Anlage zur Erzeugung steriler Luft gemäß dem Verfahren nach einem der Ansprüche 1 bis 5 mit folgenden hintereinander angeordneten Teilen:
- mindestens ein Trockenlaufkompressor (1)
- mindestens ein Behälter (b) zur Regelung der Kompressorleistung (1) und Entzug des Wassers durch Kondensierung
- ein erster Staubabscheidungsfilter (7)
- ein Adsorptions-Desorptions-Trockner (16)
- ein zweiter Staubabscheidungsfilter (18)
- drei in Serie angeordnete Koaleszenzfilter (21, 22, 23) mit bis 0,01 μm abgestufter abnehmender Filterschwelle
- ein Kaltsterilisator (26).

7. Anlage nach Anspruch 6, dadurch gekennzeichnet, daß der Trockner (16) und der Gasabscheider (17) als Aktivstoff eine Metalloxydverbindung mit Kalium-, Natrium- und Magnesiumsilikaten verwenden und die Aktivstoffmenge im Gasabscheider größer als im Trockner ist.

8. Anlage nach Anspruch 7, dadurch gekennzeichnet, daß der Gasabscheider (17) 50 % mehr Aktivstoff als der Trockner (16) enthält.

9. Anlage nach einem der Ansprüche 6 bis 8, dadurch gekennzeichnet, daß die in Serie angeordneten Koaleszenzfilter (21, 22, 23) der Bakterienabscheidungsphase ein aus mit porösem Kunststoff ummantelten Borosilikatglasfasern bestehendes Filterelement besitzen.

10. Anlage nach Anspruch 9, dadurch gekennzeichnet, daß der letzte Filter (23) der Reihenanordnung außerdem eine Aktivkohlenabsorptionsvorrichtung enthält.

11. Anlage nach einem der Ansprüche 6 bis 10, dadurch gekennzeichnet, daß der Kaltsterilisator (26) ein Filterelement mit einer rostfreien stahlgekapselten Filterpatrone mit Wasserabweisenden Borosilikatglasfasern besitzt.

12. Anlage nach einem der Ansprüche 6 bis 11, dadurch gekennzeichnet, daß sich vor dem Gasabscheider (17) eine Vorrichtung zur Umwandlung von Kohlenmonoxyd CO in Kohlendioxyd $CO_2$ (10, 11) befindet.

13. Anlage nach Anspruch 12, dadurch gekennzeichnet, daß die Umwandlungsvorrichtung von CO in $CO_2$ (10, 11) dem Trockner (16) vorgeschaltet angeordnet ist.

14. Anlage nach Anspruch 12, dadurch gekennzeichnet, daß die Umwandlungsvorrichtung von CO in $CO_2$ (10, 11) zwischen dem Kompressor (1) und dem Behälter (7) liegt.

15. Anlage nach einem der Ansprüche 6 bis 11, dadurch gekennzeichnet, daß sich hinter dem Gasabscheider (17) eine Umwandlungsvorrichtung von Kohlenmonoxyd CO in Kohlendioxyd $CO_2$ (10, 11) befindet.

16. Anlage nach Anspruch 15, dadurch gekennzeichnet, daß die Umvandlungsvorrichtung von Kohlenmonoxyd CO in Kohlendioxyd $CO_2$ (10, 11) zwischen dem zweiten Staubabscheidungsfilter (18) und dem ersten Koaleszenzfilter (21) der Bakterienabscheidungsphase angeordnet ist.

17. Anlage nach einem der Anspruche 12 bis 16, dadurch gekennzeichnet, daß die Umwandlungsvorrichtung von CO in $CO_2$ ein Katalysegerät (10) mit einem metallischen Oxydationsmittel einer Temperatur von 120 - 130° C in Verbindung mit einem Austauscher-Kondensator (11) umfaßt.

18. Anlage nach Anspruch 17, dadurch gekennzeichnet, daß das metallische Oxydationsmittel aus platinbeschichteten Pellets aus Metall oder Feuerfestmaterial besteht.

19. Anlage nach Anspruch 18, dadurch gekennzeichnet, daß diese Pellets Oberflächenunebenheiten aufweisen.

20. Anlage nach einem der Anspruche 6 bis 19, dadurch gekennzeichnet, daß das Filterelement der Staubfilter (7, 18) eine bei 1.100° C gesinterte Aluminiumoxydfilterpatrone ist.

21. Anlage nach einem der Ansprüche 6 bis 9 und 11 bis 20, dadurch gekennzeichnet, daß der zweite Koaleszenzfilter (22) der Bakterienabscheidungsphase eine Aktivkohlenabsorptionsvorrichtung enthält.

22. Anlage nach einem der Ansprüche 6 bis 21, dadurch gekennzeichnet, daß die Filter vor dem letzten Koaleszenzfilter (23) der Bakterienabscheidungsphase eine überbemessen große Filterfläche aufweisen.

**Claims**

1. A method for producing dry sterile air for medical use, starting from compressed atmospheric air which is thereafter subjected to the following treatment stages:
- a water condensation stage,
- a dust filtration stage, down to the micrometre size,
- a dessication stage,
- a stage of elimination of gases,

- a second dust filtration stage, down to the micrometre size
- a stage of elimination of bacteria,
<u>characterized</u> in that the air is compressed by means of a compressor of the dry-pistons type and in that there are used, for the dessication stage and for the gases-elimination stage, two adsorption-desorption filters, the active product of which is of the same nature, the filter for the elimination of gases being placed downstream of the dessicator filter and comprising a larger amount of active product than the latter.

2. A method according to Claim 1, characterized in that the filter for the elimination of gases contains approximately 50 % more active product than the dessicator filter.

3. A method according to any of Claims 1 - 3, characterized in that use is made in the stage of elimination of bacteria of a series of three coalescence filters having filtration thresholds decreasing down to 0.01 micrometre, followed by a low-temperature sterilizer.

4. A method according to any of Claims 1 - 3, characterized in that filtration is carried out with a small air flow velocity through all the filters down to the second coalescence filter of the bacteria elimination stage, whereupon the air flow velocity is subsequently increased through the last coalescence filter of said stage and through the sterilizer.

5. A method according to Claim 4, characterized in that the flow velocity through the last coalescence filter of the bacteria filtration stage and through the sterilizer is from one-and-a-half to three times larger than the flow velocity through the other devices of the installation.

6. An installation for the production of sterile air, using the method according to any of Claims 1 - 5, characterized in that it comprises, sequentially
- at least one compressor (1) of the dry-pistons type,
- at least one tank (b) ensuring the regulation of the outflow from the compressor (1) and the removal of water by condensation,
- a first filter (7) for the separation of dust,
- a dessicator operating by adsorption-desorption (16),
- a device for eliminating gases by adsorption-desorption (17),
- a second filter (18) for the separation of dust,
-a series of three coalescence filters (21, 22, 23) having filtration thresholds decreasing gradually down to 0.01 micrometre,
- a low-temperature separator (26).

7. An installation according to Claim 6, characterized in that the dessicator (16) and the device for the elimination of gases (17) comprise, as the active product, a compound of metal oxides with potassium, sodium and magnesium silicates, the quantity of active product contained in the device for the elimination of gases being larger than in the dessicator.

8. An installation according to Claim 7, in which the device for the elimination of gases (17) comprises 50 % more active product than the dessicator (16).

9. An installation according to any of Claims 6 - 8, characterized in that the series of coalescence filters (21, 22, 23) of the stage for the elimination of bacteria have a filtering element formed of borosilicate glass fibres coated with a sheath of porous plastic material.

10. An installation according to Claim 9, characterized in that the last filter (23) of the series further comprises an activated-carbon absorption device.

11. An installation according to any of Claims 6 - 10, characterized in that the low-temperature sterilizer (26) has a filtering element formed of a cartridge of borosilicate glass fibres having received a hydrophobic treatment, maintained inside a stainless steel frame.

12. An installation according to any of Claims 6 - 11, characterized in that it comprises, upstream of the device for eliminating gases (17), a device for converting carbon monoxide CO to carbon dioxide $CO_2$ (10, 11).

13. An installation according to Claim 12, characterized in that the device for converting CO to $CO_2$ (10, 11) is placed upstream of the dessicator (16).

14. An installation according to Claim 12, characterized in that the device for converting CO to $CO_2$ (10, 11) is placed between the compressor (1) and the tank (17).

15. An installation according to any of Claims 6 - 11, characterized in that it comprises, downstream of the apparatus for eliminating gases (17), a device for converting carbon monoxide CO to carbon dioxide $CO_2$ (10, 11).

16. An installation according to Claim 15, characterized in that the device for converting carbon monoxide CO to carbon dioxide $CO_2$ (10, 11) is placed between the second dust separation filter (18) and the first coalescence filter (21) of the bacteria filtration stage.

17. An installation according to any of Claims 12 - 16, characterized in that the device for converting CO to $CO_2$ comprises a catalysis apparatus (10) using a metallic oxidizer at a temperature from 120 to 130°C associated with an exchanger-condenser (11).

18. An installation according to Claim 17, characterized in that the metallic oxidizer is formed of platinum-coated pellets, made of metal or of a refractory material.

19. An installation according to Claim 18, characterized in that the pellets have a rough surface.

20. An installation according to any of Claims 6 - 19, characterized in that the filtering element of the dust filters (7, 18) is a cartridge of alumina vitrified at 1100°C.

21. An installation according to any of Claims 6 - 9 and 11 - 20, characterized in that the second coalescence filter (22) of the bacteria filtration stage comprises an activated carbon absorption device.

22. An installation according to any of Claims 6 - 21, characterized in that the filters located upstream of the last coalescence filter (23) of the bacteria filtration stage have an oversize passage surface.

0 123 566